# EUROPÄISCHE PATENTANMELDUNG

(11) **EP 0 602 477 A1**
(43) Veröffentlichungstag der Anmeldung: **22.06.1994**
(21) Anmeldenummer: 93119518.4
(22) Anmeldetag: 03.12.1993
(51) Int. Cl.: C07C 267/00

(54) **Verfahren zur Herstellung von aromatischen Carbodiimiden**

(30) Priorität: 16.12.1992 DE 4242504
(71) Anmelder: RHEIN-CHEMIE RHEINAU GmbH, 68219 Mannheim (DE)
(72) Erfinder: Hennig, Hans-Joachim, Dr., D-51375 Leverkusen (DE); Alter, Wolfgang, Dr., c/o BAYER S.p.A., I-20156 Milano (IT); Lüssmann, Jörg, dr., D-41539 Dormagen (DE)
(74) Vertreter: Gremm, Joachim, Dr.

(57) **Zusammenfassung**

Ein Verfahren zur Herstellung von aromatischen Carbodiimiden durch Carbodiimidisierung von aromatischen Monoisocyanaten in Gegenwart von destillierbaren organischen Phosphorverbindungen als Katalysatoren bei 120 bis 220°C bis zu einem Carbodiimidisierungsgrad von maximal 80 % und anschließende destillative Entfernung des nicht umgesetzten Ausgangsisocyanats zusammen mit dem Katalysator aus dem Reaktionsgemisch, sowie gegebenenfalls dessen anschließende destillative Aufarbeitung, sowie die nach diesem Verfahren erhältlichen Carbodiimide.

## Beschreibung

Die Erfindung betrifft ein neues Verfahren zur Herstellung von aromatischen Carbodiimiden durch Carbodiimidisierung von aromatischen Monoisocyanaten unter Verwendung von destillierbaren organischen Phosphorverbindungen als Dimerisierungskatalysatoren unter vollständiger Entfernung dieser Katalysatoren aus dem Verfahrensprodukt durch Abbruch der Carbodiimidisierungsreaktion bei einem Carbodiimidisierungsgrad von maximal 80 % und destillative Entfernung des Katalysators zusammen mit nicht umgesetztem Ausgangsisocyanat aus dem Reaktionsgemisch.

Die Herstellung von Carbodiimiden durch Carbodiimidisierung von aromatischen Monoisocyanaten in Gegenwart von organischen Phosphorverbindungen als Katalysatoren ist seit langem bekannt (vgl. z.B. US-PS 2 853 473 oder US-PS 2 853 518). Bei der Herstellung von Monocarbodiimiden werden gemäß diesen Vorveröffentlichungen im allgemeinen die zur Carbodiimidisierung führenden Reaktionsbedingungen solange aufrechterhalten, bis die eingesetzten Ausgangsisocyanate vollständig in Carbodiimide überführt sind, worauf sich eine destillative Aufarbeitung der Umsetzungsprodukte anschließt.

Die US-PS 3 502 722 beschreibt u.a die Carbodiimidisierung von 2- und/oder 6-alkylsubstituierten, d.h. sterisch gehinderten Phenylisocyanaten unter Verwendung der unterschiedlichsten Carbodiimidisierungskatalysatoren, die jedoch im allgemeinen den organischen Phosphorverbindungen der erstgenannten beiden Vorveröffentlichungen bezüglich ihrer katalytischen Wirksamkeit klar unterlegen sind. Die Phospholine und insbesondere die Phospholinoxide, hierunter besonders bevorzugt das 1-Methyl-1-oxo-phospholen, stellen besonders wirksame Carbodiimidisierungskatalysatoren dar, die eine glatte Nebenprodukt-freie Carbodiimidisierung des Ausgangsisocyanats ermöglichen. Der Nachteil der bislang unter Verwendung dieser Katalysatoren bekannt gewordenen Verfahren bestand jedoch darin, daß Katalysator-freie Carbodiimide nicht hergestellt werden konnten, da eine destillative Trennung der Verfahrensprodukte von den in geringen Konzentrationen eingesetzten Katalysatoren nicht möglich war. Andererseits werden aromatische Carbodiimide als Alterungsschutzmittel für Polyester- und Polyester-Polyurethan-Kunststoffe eingesetzt, ein Einsatzgebiet, das einen hohen Reinheitsgrad der aromatischen Carbodiimide und somit im Falle der Verwendung der genannten Phosphorverbindungen als Katalysator deren quantitative Entfernung aus dem Carbodiimid erfordert.

Es war daher die der Erfindung zugrunde liegende Aufgabe, ein neues Verfahren zur Herstellung von aromatischen Carbodiimiden durch Carbodiimidisierung von aromatischen Monoisocyanaten unter Verwendung der bevorzugten Phosphorverbindungen des Standes der Technik als Katalysator zur Verfügung zu stellen, welches eine praktisch vollständige Entfernung des eingesetzten Katalysators aus dem Verfahrensprodukt durch eine einfache Maßnahme gestattet.

Diese Aufgabe konnte mit der Bereitstellung des nachstehend näher beschriebenen erfindungsgemäßen Verfahrens gelöst werden.

Gegenstand der Erfindung ist ein Verfahren zur Herstellung von aromatischen Carbodiimiden durch Carbodiimidisierung von aromatischen Monoisocyanaten in Gegenwart von destillierbaren, die Carbodiimidisierung von Isocyanaten unter Kohlendioxidabspaltung beschleunigenden organischen Phosphorverbindungen als Katalysatoren bei 120 bis 220°C, dadurch gekennzeichnet, daß man bei einem Carbodiimidisierungsgrad der als Ausgangsmaterial eingesetzten Monoisocyanate von maximal 80 % das noch nicht umgesetzte Ausgangsisocyanat zusammen mit dem Katalysator destillativ aus dem Reaktionsgemisch entfernt und das Carbodiimid als Destillationsrückstand dieser Destillation isoliert.

Ausgangsmaterialien für das erfindungsgemäße Verfahren sind beliebige, destillierbare Monoisocyanate mit aromatisch gebundenen Isocyanatgruppen. Vorzugsweise werden in 2- und/oder 6-Stellung zur Isocyanatgruppe alkylsubstituierte und gegebenenfalls noch andere Substituenten tragende Phenylisocyanate der in US-PS 3 502 722, Kolonne 2, Zeile 55 - Kolonne 3, Zeile 15, genannten Art eingesetzt, wobei auch erfindungsgemäß das 2,6-Diisopropyl-phenylisocyanat das besonders bevorzugte Ausgangsmaterial darstellt.

Als Katalysatoren werden beim erfindungsgemäßen Verfahren destillierbare, die Carbodiimidisierung von aromatischen Isocyanaten beschleunigende organische Phosphorverbindungen, insbesondere Phospholine oder Phospholinoxide eingesetzt. Geeignet sind beispielsweise die in US-PS 2 853 518 genannten Phospholine oder Phospholidine und insbesondere die in US-PS 2 853 473 genannten Phospholinoxide, wobei erfindungsgemäß 1-Methyl-1-oxo-phopholen besonders bevorzugt ist. Die Katalysatoren werden bei der Durchführung des erfindungsgemäßen Verfahrens im allgemeinen in einer Menge von 50 bis 1000 ppm (Gewicht), bezogen auf das Gewicht des Ausgangsisocyanats, eingesetzt. Der Katalysator kann dem Ausgangsisocyanat zu Beginn der Reaktion oder teilweise auch im Verlauf der Reaktion in Portionen einverleibt werden. Die angegebene Menge bezieht sich auf die Gesamtmenge des eingesetzten Katalysators.

Zur Durchführung des erfindungsgemäßen Verfahrens wird in der ersten Verfahrensstufe das Katalysator-haltige Ausgangsisocyanat, gegebenenfalls unter portionsweiser Zugabe weiterer Katalysatormengen, auf die Reaktionstemperatur von 120 bis 220, vorzugsweise 160 bis 180°C erhitzt, bis maximal 80, vorzugsweise 50 bis 70 % der Isocyanatgruppen des Ausgangsisocyanats unter Carbodiimidisierung abreagiert haben, was leicht an der Menge des abgespaltenen Kohlendioxids zu erkennen ist. Während dieser ersten Verfahrensstufe werden die Druckbedingungen so eingestellt, daß ein Abdestillieren von Ausgangsisocyanat unterbleibt. Hierzu ist es im allgemeinen ausreichend, unter Normaldruck zu arbeiten. Die Umsetzung erfolgt vorzugsweise kontinuierlich, beispielsweise in einer Rührkesselkaskade.

Die zweite Stufe des erfindungsgemäßen Verfahrens besteht darin, das nicht umgesetzte Monoisocyanat zusammen mit dem Katalysator destillativ aus dem Reaktionsgemisch zu entfernen. Der erfindungswesentliche Punkt besteht darin, daß das hierbei abdestillierende, nicht umgesetzte Ausgangsisocyanat als Schleppmittel für den Katalysator dient. Diese destillative Entfernung von Ausgangsisocyanat und Katalysator erfolgt im allgemeinen innerhalb des Temperaturbereichs von 150 bis 220°C unter einem Betriebsdruck von 1 bis 50 mbar in einer kontinuierlich betriebenen Destillationsanlage, beispielsweise in einer Füllkörperkolonne, wobei für möglichst kurze Sumpfverweilzeiten des Katalysator enthaltenden Reaktionsgemischs Sorge zu tragen ist. Bei Verweilzeiten im Minutenbereich wird keine Störung der Destillation beobachtet.

Als Destillat fällt in der zweiten Reaktionsstufe ein Gemisch aus Ausgangsisocyanat und Katalysator an, welches an den Prozeßanfang zurückgeführt werden kann. Das als Sumpfprodukt anfallende rohe Verfahrensprodukt kann gewünschtenfalls einer weiteren Feindestillation unterzogen werden, wobei das Carbodiimid als Destillat von höher siedenden Verunreinigungen befreit wird. Diese destillative Reindarstellung des Verfahrensprodukts erfolgt vorzugsweise kontinuierlich unter Verwendung eines Dünnschichtverdampfers innerhalb des Temperaturbereichs von 150 bis 250°C und innerhalb des Druckbereichs von 0,5 bis 50 mbar. Als Destillat werden hierbei farblose Carbodiimide mit einem Phosphor-Gehalt von unter 5 ppm (Gewicht) erhalten.

Die erfindungsgemäßen Verfahrensprodukte stellen wertvolle Hydrolysestabilisatoren für Estergruppen aufweisende Kunststoffe dar.

Die nachfolgenden Beispiele dienen der weiteren Erläuterung des erfindungsgemäßen Verfahrens.

### Beispiel 1

### 1. Verfahrensstufe:

### Partielle Carbodiimidisierung von 2,6-Diisopropyl-phenylisocyanat

In eine kontinuierlich betriebene Reaktorkaskade, bestehend aus 4 gerührten Reaktoren mit einem Füllvolumen von jeweils 15 l werden mit Hilfe von Dosierpumpen stündlich 15 kg 2,6-Diisopropyl-phenylisocyanat und 0,0028 kg 1-Methyl-1-oxo-phospholen eindosiert Die Reaktionstemperatur beträgt 175°C, die mittlere Verweilzeit 4 Stunden. Nach Austritt aus der Reaktorkaskade wird das heiße Gemisch aus 2,2',6,6'-Tetraisopropyl-diphenylcarbodiimid, 2,6-Diisopropyl-phenylisocyanat und 1-Methyl-1-oxo-phospholen auf Raumtemperatur abgekühlt. Während der Isocyanatgruppen-Gehalt von reinem 2,6-Diisopropyl-phenylisocyanat 20,7 % beträgt, hat das Reaktionsgemisch einen NCO-Gehalt von 7,1 %. Das Reaktionsgemisch enthält somit 34,3 Gew.-% 2,6-Diisopropyl-phenylisocyanat und 65,7 % Gew.-% Carbodiimid.

### 2. Verfahrensstufe:

### Destillative Edukt- und Katalysator-Abtrennung

In eine Destillationskolonne mit drei Kolonnenschüssen (Länge jeweils 500 mm, Innendurchmesser 80 mm mit SULZER BX Gewebepackung), deren Kolonnensumpf auf 200°C erhitzt wird, werden bei einem Betriebsdruck von 0,3 mbar am Kolonnenkopf stündlich 14 kg auf 105°C vorgewärmtes Gemisch zwischen dem zweiten und dritten Schuß eingetragen. Bei einem Kolonnensumpfvolumen von 2,06 l beträgt die mittlere Sumpfverweilzeit 8,8 min. Stündlich fallen 8 kg Carbodiimid als Sumpfablauf und 6 kg Destillat, das den Katalysator 1-Methyl-1-oxo-phospholen enthält, an.

### 3. Verfahrensstufe:

### Destillative Aufarbeitung des Carbodiimids

In einem Dünnschichtverdampfer (⌀ 160 mm, Heizfläche 1,5 m²) werden bei einer Betriebstemperatur von 180°C und einem Betriebsdruck von 0,9 mbar stündlich 22 kg rohes Carbodiimid aus Verfahrensstufe 2 eindosiert. Als Kopfprodukt erhält man stündlich 21,5 kg reinstes 2,2',6,6'-Tetraisopropyl-diphenylcarbodiimid mit einem Carbodiimidgehalt von 10,8 % und einer APHA-Farbzahl von 230. Der Phosphor-Gehalt liegt unter 5 ppm (Gewicht).

### Beispiel 2

In einem gerührten Reaktor mit einem Volumen von 2000 l werden 1180,6 kg, 2,6-Diisopropyl-phenylisocyanat und 0,5 kg 1-Methyl-1-oxo-phospholen allmählich bis auf 165°C erhitzt. Mittels einer Gasuhr in der Abgasleitung des Reaktors läßt sich die Menge des Kohlendioxids, das sich beim Carbodiimidisierungsprozeß als Kuppelprodukt bildet, erfassen. Nach Abspaltung von 41,1 m³ CO₂ (unter Normalbedingungen), d.h. nach ca. 48 Stunden, wird der Carbodiimidisierungsprozeß durch Abkühlen des Reaktorinhaltes unterbrochen. Der Isocyanatgruppen-Gehalt des Reaktionsgemisches beträgt 8,2 %, der Anteil an nicht umgesetztem 2,6-Diisopropyl-phenylisocyanat somit 39,6 Gew.-%.

Die destillative Aufarbeitung (2. Verfahrensstufe) und destillative Reindarstellung des Verfahrensprodukts (3. Verfahrensstufe) erfolgten in Analogie zu Beispiel 1. Es resultiert ein praktisch farbloses Carbodiimid mit einem Phosphor-Gehalt von unter 3 ppm.

## Patentansprüche

1. Verfahren zur Herstellung von aromatischen Carbodiimiden durch Carbodiimidisierung von aromatischen Monoisocyanaten in Gegenwart von destillierbaren, die Carbodiimidisierung von Isocyanaten unter Kohlendioxidabspaltung beschleunigenden organischen Phosphorverbindungen als Katalysatoren bei 120 bis 220°C, dadurch gekennzeichnet, daß man bei einem Carbodiimidisierungsgrad der als Ausgangsmaterial eingesetzten Monoisocyanate von maximal 80 %, das noch nicht umgesetzte Ausgangsisocyanat zusammen mit dem Katalysator destillativ aus dem Reaktionsgemisch entfernt und das Carbodiimid als Destillationsrückstand dieser Destillation isoliert.

2. Verfahren gemäß Anspruch 1, dadurch gekennzeichnet, daß man das als Destillat isolierte Gemisch aus Ausgangsisocyanat und Katalysator in den Carbodiimidisierungsprozeß zurückführt.

3. Verfahren gemäß Anspruch 1, dadurch gekennzeichnet, daß man das als Destillationsrückstand gewonnene Verfahrensprodukt einer destillativen Reindestillation zuführt.

4. Verfahren gemäß Anspruch 1 bis 3, dadurch gekennzeichnet, daß man als Ausgangsisocyanat 2- und/oder 6-alkylsubstituiertes Phenylisocyanat verwendet.

5. Verfahren gemäß Anspruch 1 bis 4, dadurch gekennzeichnet, daß man als Ausgangsisocyanat 2,6-Diisopropyl-phenylisocyanat verwendet.

6. Verfahren gemäß Anspruch 1 bis 5, dadurch gekennzeichnet, daß man als Katalysator 1-Methyl-1-oxo-phospholen verwendet.

7. Gemäß Anspruch 1 bis 6 erhältliche Carbodiimide.
